# EUROPÄISCHE PATENTSCHRIFT

(11) **EP 0 661 277 B1**
(45) Veröffentlichungstag und Bekanntmachung des Hinweises auf die Patenterteilung: **11.03.1998**
(21) Anmeldenummer: 94117519.2
(22) Anmeldetag: 07.11.1994
(51) Int. Cl.: C07D 249/12

(54) **Verfahren und neue Zwischenprodukte zur Herstellung von Triazolinonen**
Process for making triazolines and new untermediates used therein
Procédé et nouveaux intermédiaires pour la préparation de triazolines

(30) Priorität: 18.11.1993 DE 4339412
(43) Veröffentlichungstag der Anmeldung: 05.07.1995
(73) Patentinhaber: BAYER AG, 51368 Leverkusen (DE)
(72) Erfinder: Linker, Karl-Heinz, D-51377 Leverkusen (DE); Haas, Wilhelm, Dr., D-50259 Pulheim (DE); Findeisen, Kurt, Prof. Dr., D-51375 Leverkusen (DE); Diehr, Hans-Joachim, Dr., D-42329 Wuppertal (DE)

(56) Entgegenhaltungen:
- EP-A- 0 139 183
- EP-A- 0 276 793
- EP-A- 0 495 226
- US-A- 3 780 052
- ORG. MASS SPECTROM., Bd. 12, no.10, 1977 FRANCE, Seiten 638-643, & CHEMICAL ABSTRACTS, vol. 88, no. 17, 24.April 1978 Columbus, Ohio, US; abstract no. 120169a, AOUIAL 'Fragmentation under electron impact of 4,5-diamino-1,2,4-triazoles and 4-amino-5-mercapto-1,2,4-triazoles.' Seite 461; Spalte 2;

## Beschreibung

Die Erfindung betrifft ein neues Verfahren und neue Zwischenprodukte zur Herstellung von weitgehend bekannten Triazolinonen, welche als Zwischenprodukte zur Herstellung von Herbiziden und Insektiziden verwendet werden können.

Es ist bekannt, daß man bestimmte substituierte Triazolinone, wie z.B. die Verbindung 4-Methyl-5-trifluormethyl-2,4-dihydro-3H-1,2,4-triazol-3-on erhält, wenn man entsprechende Triazolinthione, wie z.B. die Verbindung 4-Methyl-5-trifluormethyl-2,4-dihydro-3H-1,2,4-triazol-3 -thion, zunächst mit einem Alkylierungsmittel, wie z.B. Methyliodid, in Gegenwart eines Säurebindemittels, wie z.B. Natriummethylat umsetzt, das hierbei gebildete Alkylthiotriazolderivat auf übliche Weise isoliert, dann mit Hydrogenperoxid in Gegenwart von Essigsäure erhitzt, nach Abkühlen neutralisiert und aufübliche Weise aufarbeitet (vgl. US-P 3780052 - Example 2).
Weiter ist bekannt, daß die oben genannte Verbindung 4-Methyl-5-trifluormethyl-2,4-dihydro-3H-1,2,4-triazol-3-on auch durch Erhitzen von 1-Trifluoracetyl-4-methylsemicarbazid auf 160°C bis 180°C, anschließende Extraktion mit Essigsäureethylester und Säulenchromatographie erhalten werden kann (vgl. US-P 3780052 - Example 3).

In EP-A-0 495 226 und EP-A-0 276 793 werden 3-Aryl-5-alkylthio-4H-1,2,4-triazole als Pharmazeutika beschrieben. In der EP-A-0 139 183 werden Alkylthiotriazole in herbiziden Mitteln eingesetzt. Schließlich wird in Org. Mass. Spectrom, Bd. 12, No. 10, 1977, auf S. 638 die Verbindung 4-Amino-3-methyl-5-methylthio-triazol beschrieben.

Bei den beiden angegebenen Synthesemethoden sind jedoch Ausbeute und Qualität der erhaltenen Produkte sehr unbefriedigend.

Gegenstand der vorliegenden Anmeldung ist ein neues Verfahren zur Herstellung von Triazolinonen der allgemeinen Formel (I) 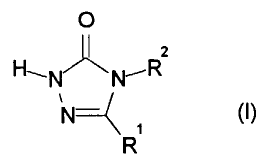 in welcher
- R¹: für durch Halogen, Cyano oder C₁-C₄-Alkoxy substituiertes Alkyl mit 1 bis 6 Kohlenstoffatomen steht und
- R²: für Amino, für jeweils gegebenenfalls durch Halogen, Cyano oder C₁-C₄-Alkoxy substituiertes Alkyl, Alkenyl, Alkinyl, Alkoxy, Alkylamino oder Dialkylamino mit jeweils bis zu 6 Kohlenstoffatomen in den Alkyl-, Alkenyl- oder Alkinylgruppen oder für jeweils gegebenenfalls durch Halogen, Cyano, C₁-C₄-Alkyl, C₁-C₄-Alkoxy oder C₁-C₄-Alkoxy-carbonyl substituiertes C₃-C₆-Cycloalkyl, C₃-C₆-Cycloalkyl-C₁-C₂-alkyl oder Phenyl steht,
dadurch gekennzeichnet, daß man
Alkylsulfonyltriazolderivate der allgemeinen Formel (II) 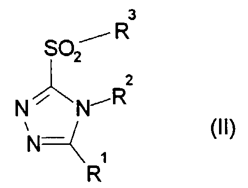 in welcher
- R¹ und R²: die oben angegebenen Bedeutungen haben und
- R³: für Alkyl mit 1 bis 4 Kohlenstoffatomen steht,
mit wässriger Alkalilauge bei Temperaturen zwischen 0°C und 100°C bei Normaldruck umsetzt und nach Ansäuern auf übliche Weise aufarbeitet.

Überraschenderweise können nach dem erfindungsgemäßen Verfahren die Triazolinone der allgemeinen Formel (I) auf einfache Weise in sehr hohen Ausbeuten - welche im Vergleich mit dem Stand der Technik erheblich verbessert sind - und in hoher Reinheit erhalten werden.

Das erfindungsgemäße Verfahren stellt somit eine wertvolle Bereicherung des Standes der Technik dar.

Nach dem erfindungsgemäßen Verfahren werden vorzugsweise Verbindungen der Formel (I) hergestellt, in welcher
- R¹: für jeweils durch Fluor, Chlor, Brom, Cyano, Methoxy oder Ethoxy substituiertes Methyl, Ethyl, n- oder i-Propyl, n-, i- oder s-Butyl steht und
- R²: für Amino, für jeweils gegebenenfalls durch Fluor, Chlor, Cyano, Methoxy oder Ethoxy substituiertes Methyl, Ethyl, n- oder i-Propyl, n-, i- oder s-Butyl, Allyl, Propargyl, Methoxy, Ethoxy, n- oder i-Propoxy, n-, i- oder s-Butoxy, Methylamino, Ethylamino, n- oder i-Propylamino, n-, i- oder s-Butylamino, Dimethylamino oder Diethylamino oder für jeweils gegebenenfalls durch Fluor, Chlor, Brom, Cyano, Methyl, Ethyl, n- oder i-Propyl, Methoxy-carbonyl oder Ethoxy-carbonyl substituiertes Cyclopropyl, Cyclobutyl, Cyclopentyl, Cyclohexyl oder Phenyl steht.

Die bei den Restedefinitionen genannten Kohlenwasserstoffreste, wie Alkyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Eine ganz besonders bevorzugte Gruppe von Verbindungen, welche nach dem erfindungsgemäßen Verfahren hergestellt werden können, sind die Verbindungen der Formel (I), in welcher
- R¹: für jeweils entsprechend einfach, zweifach, dreifach, vierfach, fünffach, sechsfach oder siebenfach durch Fluor und/oder Chlor substituiertes Methyl, Ethyl, n-oder i-Propyl steht und
- R²: für Methyl, Ethyl, n- oder i-Propyl, Methoxy, Ethoxy, Methylamino, Ethylamino, Dimethylamino, Cyclopropyl, Phenyl oder Tolyl steht.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Verwendet man beispielsweise 3-Difluormethyl-4-methyl-5-methylsulfonyl-4H-1,2,4-triazol und Kaliumhydroxid als Ausgangsstoffe, so kann der Reaktionsablauf beim erfindungsgemäßen Verfahren durch das folgende Formelschema skizziert werden:

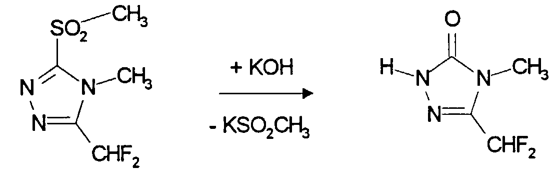

Die beim erfindungsgemäßen Verfahren zur Herstellung der Verbindungen der allgemeinen Formel (I) als Ausgangsstoffe zu verwendenden Alkylsulfonyltriazolderivate sind durch die Formel (II) allgemein definiert.

In der Formel (II) haben R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹ und R² angegeben wurden; R³ steht vorzugsweise für Methyl oder Ethyl.

Die Ausgangsstoffe der allgemeinen Formel (II) sind noch nicht aus der Literatur bekannt; sie sind als neue Stoffe Gegenstand der vorliegenden Anmeldung.

Man erhält die neuen Alkylsulfonyltriazolderivate der allgemeinen Formel (II), wenn man Alkylthiotriazolderivate der allgemeinen Formel (III) 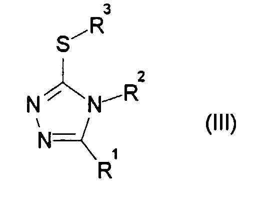 in welcher
- R¹, R² und R³: die oben angegebenen Bedeutungen haben,
mit Oxidationsmitteln, gegebenenfalls in Gegenwart von Reaktionshilfsmitteln und gegebenenfalls in Gegenwart von Verdünnungsmitteln, bei Temperaturen zwischen 0°C und 100°C umsetzt (vgl. die Herstellungsbeispiele).

Geeignete Oxidationsmittel sind hierbei beispielsweise Sauerstoff, Ozon, Hydrogenperoxid, Chlor, Chlorlauge, Kaliumpermanganat, Perameisensäure, Peressigsäure, Perpropionsäure sowie gegebenenfalls halogenierte Perbenzoesäuren.

Geeignete Reaktionshilfsmittel - insbesondere bei Einsatz von Hydrogenperoxid - sind hierbei vor allem Salze von Metallen der IV., V. und VI. Nebengruppe des Periodensystems der Elemente. Als Beispiele hierfür seien Natrium(meta)vanadat, Natriummolybdat und Natriumwolframat genannt.

Geeignete Verdünnungsmittel sind hierbei insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methyl-formanilid, N-Methyl-pyrrolidon oder Hexamethylphosphorsäuretriamid; Carbonsäuren wie Ameisensäure, Essigsäure oder Propionsäure, Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die als Vorprodukte benötigten Alkylthiotriazolderivate der allgemeinen Formel (III) sind mit Ausnahme der Verbindung 4-Methyl-3-trifluormethyl-5-methylthio-1H-1,2,4-triazol (vgl. US-P 3780052 - Example 2) noch nicht aus der Literatur bekannt; sie sind als neue Stoffe Gegenstand der vorliegenden Anmeldung.

Man erhält die Alkylthiotriazolderivate der allgemeinen Formel (III), wenn man Triazolinthione der allgemeinen Formel (IV) 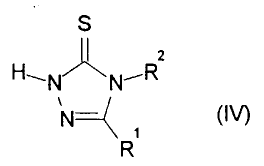 in welcher
- R¹ und R²: die oben angegebenen Bedeutungen haben,
mit Alkylierungsmitteln, wie z.B. Methylbromid, Methyliodid, Ethylbromid oder Ethyliodid, gegebenenfalls in Gegenwart eines Säureakzeptors, wie z.B. Natrium- oder Kalium-hydroxid, und gegebenenfalls in Gegenwart eines Verdünnungsmittels, wie z.B. Methanol oder Ethanol, bei Temperaturen zwischen 0°C und 100°C umsetzt (vgl. die Herstellungsbeispiele).

Die Triazolinthione der allgemeinen Formel (IV) sind bekannt und/oder können nach an sich bekannten Verfahren hergestellt werden (vgl. US-P 3719686 und US-P 3780052).

Man erhält die Triazolinthione der allgemeinen Formel (IV), wenn man Carbonsäuren der allgemeinen Formel (V)

R¹-CO-X (V)

in welcher
- R¹: die oben angegebene Bedeutung hat und
- X: für Hydroxy oder Halogen steht,
mit Alkylthiosemicarbaziden der allgemeinen Formel (VI)

R²-NH-CS-NH-NH₂ (VI)

in welcher
- R²: die oben angegebene Bedeutung hat,
bei Temperaturen zwischen 0°C und 100°C umsetzt (vgl. die Herstellungsbeispiele).

Das erfindungsgemäße Verfahren zur Herstellung von Triazolinonen der allgemeinen Formel (I) wird unter Einsatz wässriger Alkalilaugen durchgeführt. Vorzugsweise kommen hierbei wässrige Lösungen von Natriumhydroxid oder von Kaliumhydroxid in Betracht.

Die Reaktionstemperaturen können bei der Durchführung des erfindungsgemäßen Verfahrens in einem größeren Bereich variiert werden. Im allgemeinen arbeitet man bei Temperaturen zwischen 0°C und 100°C, vorzugsweise bei Temperaturen zwischen 10°C und 80°C, insbesondere bei Temperaturen zwischen 20°C und 60°C.

Das erfindungsgemäße Verfahren wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens zur Herstellung der Verbindungen der Formel (I) setzt man pro Mol des Alkylsulfonyltriazolderivates der Formel (II) im allgemeinen 1 bis 15 Mol, vorzugsweise 2 bis 8 Mol eines in Wasser gelösten Alkalimetallhydroxids ("Alkalilauge") ein.

In einer bevorzugten Ausführungsform des erfindungsgemäßen Verfahrens wird die Alkalilauge vorgelegt und das Alkylsulfonyltriazol der Formel (II) - gegebenenfalls unter Kühlen - langsam eindosiert. Dann wird das Reaktionsgemisch - gegebenenfalls bei erhöhter Temperatur - bis zum Ende der Umsetzung gerührt. Anschließend wird mit einer Säure, vorzugsweise einer Protonensäure wie z.B. Salzsäure oder Schwefelsäure angesäuert. Vor oder nach dem Ansäuern kann gegebenenfalls das Reaktionsgemisch aufkonzentriert oder mit Wasser verdünnt werden.

Die Aufarbeitung und Isolierung des Reaktionsproduktes kann auf übliche Weise durchgeführt werden. Beispielsweise wird mit einem mit Wasser praktisch nicht mischbaren organischen Lösungsmittel, wie z.B. Methylenchlorid oder Essigsäureethylester geschüttelt, die organische Phase abgetrennt, und - beispielsweise mit Natriumsulfat - getrocknet. Dann wird filtriert und vom Filtrat das Lösungsmittel unter vermindertem Druck sorgfältig abdestilliert. Das als Rückstand verbleibende Rohprodukt kann dann direkt für weitere Umsetzungen eingesetzt, oder auf übliche Weise, beispielsweise durch Säulenchromatographie und/oder Umkristallisation weiter gereinigt werden.

Die nach dem erfindungsgemäßen Verfahren herzustellenden Triazolinone der Formel (I) können als Zwischenprodukte zur Herstellung von landwirtschaftlich nutzbaren Wirkstoffen verwendet werden (vgl. US-P 3780052, US-P 3780053, US-P 3780054 und EP-A 341489).

### Herstellungsbeispiele:

### Beispiel 1

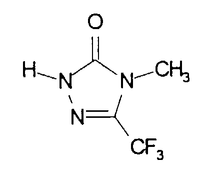

112 g (0,49 Mol) 4-Methyl-3-methylsulfonyl-5-trifluormethyl-4H-1,2,4-triazol werden zu einer Lösung von 111 g (2,78 Mol) Natriumhydroxid in 1,64 Liter Wasser gegeben und das Reaktionsgemisch wird 16 Stunden bei 50°C gerührt. Die hierbei entstandene klare, gelbliche Lösung wird anschließend mit konz. Salzsäure angesäuert, dann im Wasserstrahlvakuum auf etwa das halbe Volumen eingeengt und schließlich mit Methylenchlorid durchgeschüttelt. Nach Abtrennen der organischen Phase wird die wässrige Phase noch zweimal mit Methylenchlorid nachextrahiert. Die vereinigten organischen Phasen werden mit Natriumsulfat getrocknet und filtriert. Vom Filtrat wird dann das Lösungsmittel im Wasserstrahlvakuum sorgfältig abdestilliert.

Man erhält 71 g (87 % der Theorie) 4-Methyl-5-trifluormethyl-2,4-dihydro-3H-1,2,4-triazol-3-on als weißes Pulver vom Schmelzpunkt 64°C.

### Beispiel 2

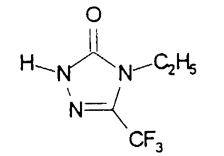

317 g (1,30 Mol) 4-Ethyl-3-trifluormethyl-5-methylsulfonyl-4H-1,2,4-triazol werden bei maximal 30°C (Kühlung mit Eiswasser) zu einer Lösung von 294 g (7,35 Mol). Natriumhydroxid in 1,8 Liter Wasser getropft und 8 Stunden bei 20°C gerührt. Anschließend wird auf 1,5 Liter Eiswasser ausgerührt, mit konz. Salzsäure auf pH 2-3 eingestellt, mit Essigsäureethylester mehrfach extrahiert, über Natriumsulfat getrocknet und das Lösungsmittel abgedampft.

Man erhält 215 g (91% der Theorie) 4-Ethyl-5-trifluormethyl-2,4-dihydro-3H-1,2,4-triazol-3-on vom Schmelzpunkt 54°C.

Analog können beispielsweise auch die in der nachstehenden Tabelle 1 aufgeführten Verbindungen der Formel (I) hergestellt werden.

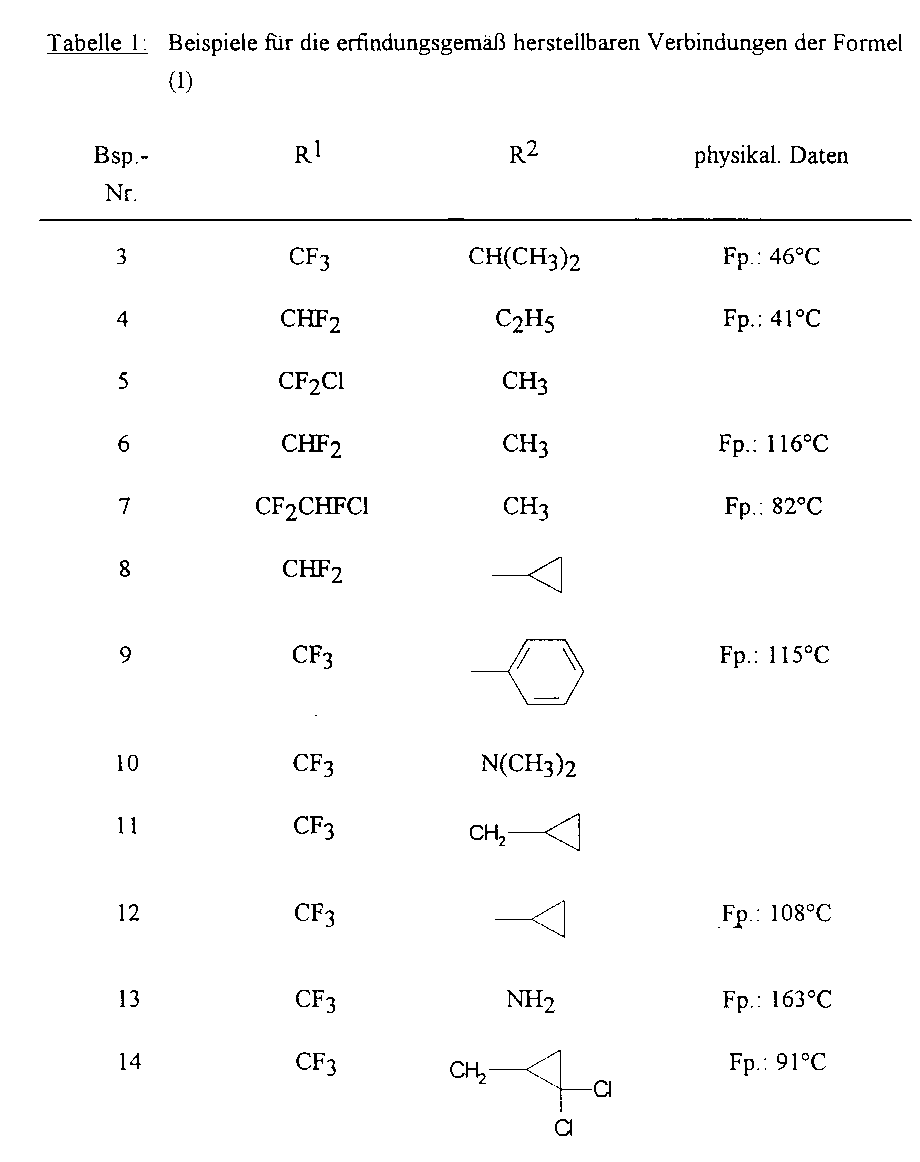

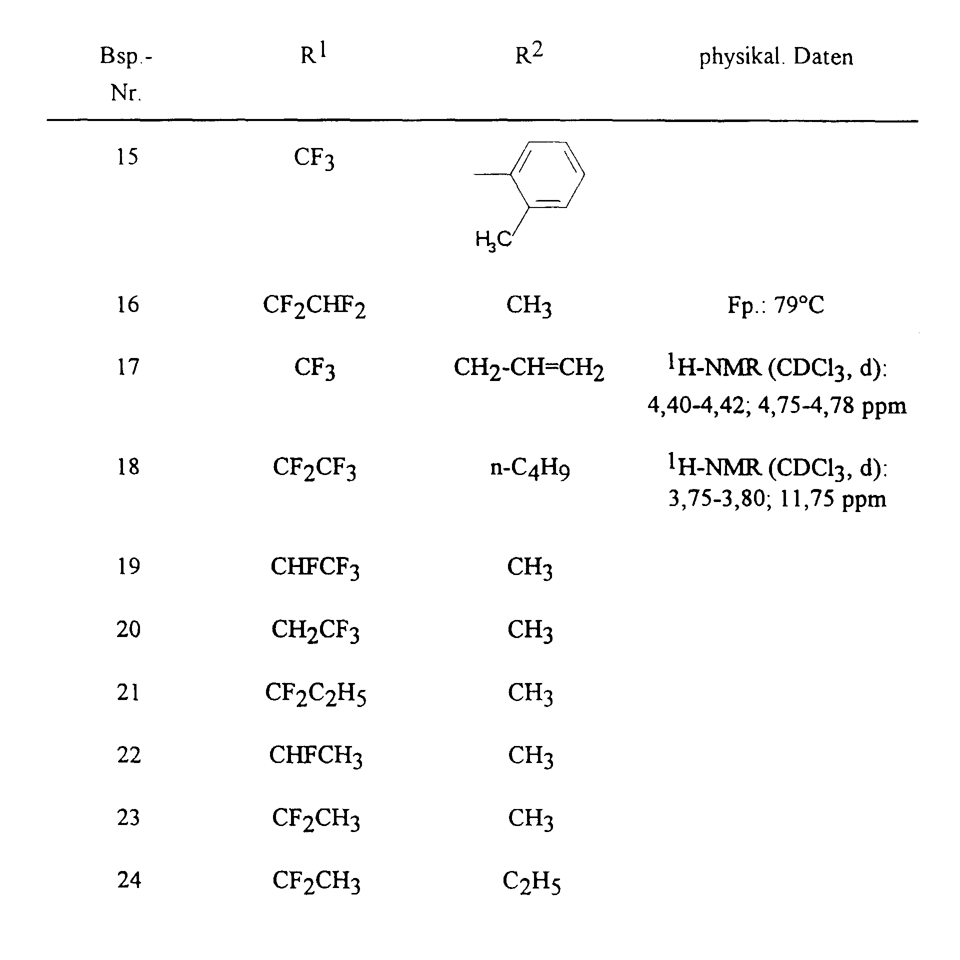

### Ausgangsstoffe der Formel (II):

### Beispiel (II-1):

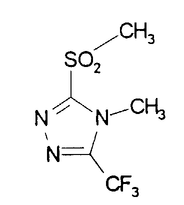

335 g (1,7 Mol) 4-Methyl-3-trifluormethyl-5-methylthio-4H-1,2,4-triazol werden in 2,5 Liter Methylenchlorid vorgelegt. Dazu werden 5 g (4 mMol) Ammoniumheptamolybdat-Tetrahydrat und 225 g (4,9 Mol) Ameisensäure gegeben, die Mischung wird dann zum Rückfluß erhitzt und dabei werden unter starkem Rühren 560 g einer 35%igen Perhydrol-Lösung (5,76 Mol H₂O₂) tropfenweise dazugegeben. Das Erhitzen unter Rückfluß wird noch 16 Stunden fortgesetzt; dann wird überschüssiges Hydrogenperoxid mit Natriumhydrogensulfit (39%ige wäßrige Lösung) beseitigt und das Produkt dreimal mit Methylenchlorid extrahiert. Die vereinigten organischen Phasen werden mit 5%iger wäßriger Natriumhydrogensulfit-Lösung und dann mit Wasser gewaschen, über Magnesiumsulfat getrocknet und filtriert.

Vom Filtrat wird das Lösungsmittel im Wasserstrahlvakuum sorgfältig abdestilliert.

Man erhält 325 g (83,5% der Theorie) 4-Methyl-3-methylsulfonyl-5-tritluormethyl-4H-1,2,4-triazol vom Schmelzpunkt 96°C.

### Beispiel (II-2)

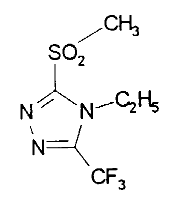

727,6 g (3,45 Mol) 4-Ethyl-3-trifluormethyl-5-methylthio-4H-1,2,4-triazol, 624 g Ameisensäure und 10 g Ammoniummolybdat werden in 2 Liter Dichlormethan vorgelegt. Bei maximal 35°C werden innerhalb einer Stunde 2346 g 30%ige Wasserstoffperoxid-Lösung zugetropft. Man rührt 8 Stunden nach (anfangs noch exotherm), versetzt mit 2 Liter Eiswasser, beseitigt durch Zugabe von Natriumhydrogensulfit-Lösung überschüssiges Wasserstoffperoxid, extrahiert mehrfach mit Dichlormethan, trocknet die vereinigten organischen Phasen über Natriumsulfat und verdampft das Lösungsmittel am Rotationsverdampfer.

Man erhält 811,6 g (96,8% der Theorie) 4-Ethyl-3-trifluormethyl-5-methylsulfonyl-4H-1,2,4-triazol als Öl.

¹H-NMR (CDCl₃, d): 1,56-1,61; 3,60; 4,48-4,56 ppm.

Analog können beispielsweise auch die in der nachstehenden Tabelle 2 aufgeführten Verbindungen der Formel (II) hergestellt werden.

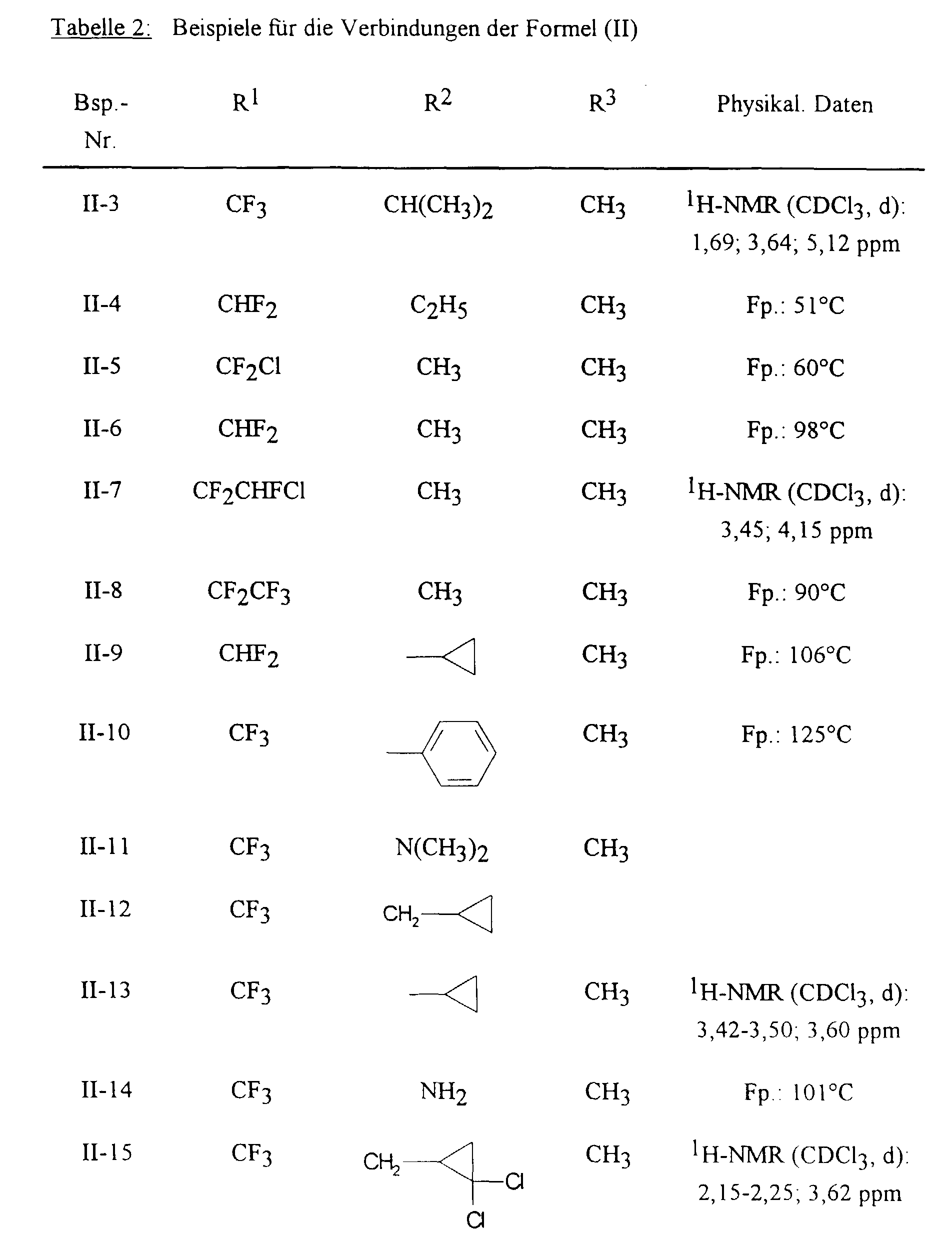

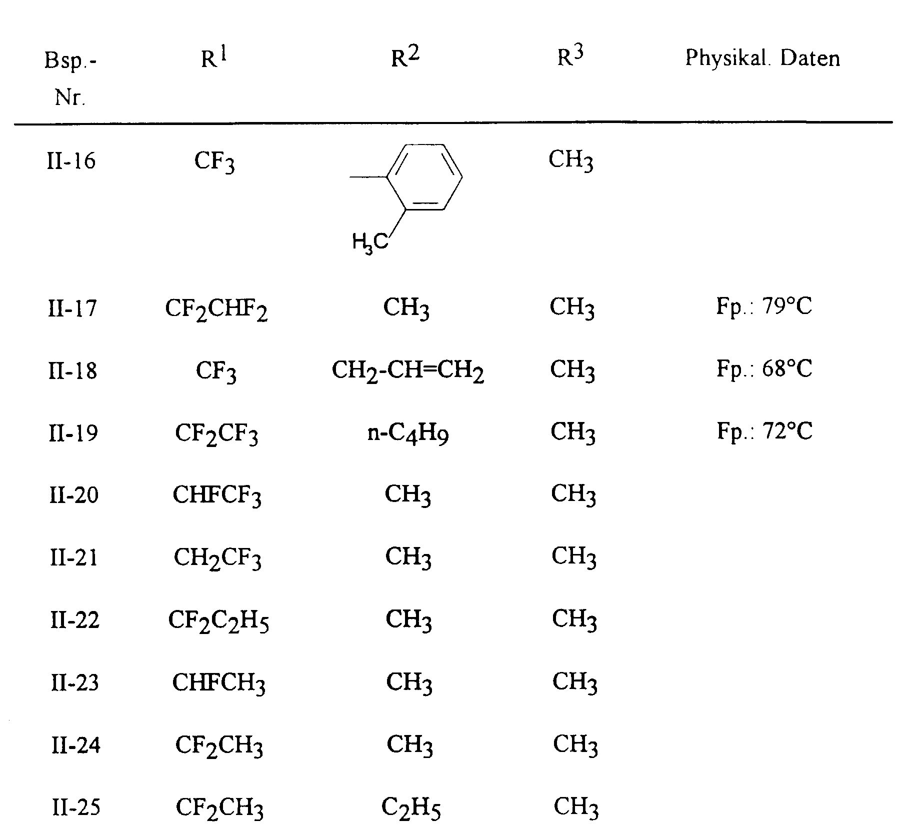

### Vorprodukte der Formel (III):

### Beispiel (III-1)

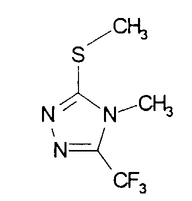

250 g (1,36 Mol) 4-Methyl-5-trifluormethyl-2,4-dihydro-3H-1,2,4-triazol-3-thion werden in 1,64 Liter Ethanol vorgelegt. Dazu werden 61,2 g (1,53 Mol) Natriumhydroxid gegeben, woraufhin sich nach exothermer Reaktion innerhalb einer Stunde eine klare Lösung bildet. Anschließend werden 217 g (1,53 Mol) Methyliodid tropfenweise dazugegeben und die Mischung 16 Stunden bei 20°C gerührt. Dann wird auf etwa das halbe Volumen eingeengt, mit ca. 3 Liter Eiswasser verrührt, mit konz. Salzsäure angesäuert und dreimal mit Methylenchlorid extrahiert. Die vereinigten organischen Phasen werden über Magnesiumsulfat getrocknet und filtriert. Vom Filtrat wird das Lösungsmittel im Wasserstrahlvakuum sorgfältig abdestilliert.

Man erhält 207,4 g (77,4% der Theorie) 4-Methyl-3-methylthio-5-trifluormethyl-4H-1,2,4-triazol als gelbes Pulver vom Schmelzpunkt 46°C.

### Beispiel (III-2)

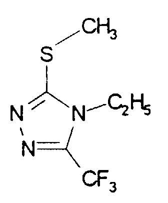

371,3 g (1,88 Mol) 4-Ethyl-5-trifluormethyl-2,4-dihydro-3H-1,2,4-triazolin-3-thion und 83,5 g (2,09 Mol) Natriumhydroxid werden in 1,5 Liter Ethanol vorgelegt. Bei maximal 30°C (Kühlung) tropft man 296,8 g (2,09 Mol) Jodmethan zu und rührt 8 Stunden bei 20°C nach. Die Reaktionslösung wird mit 1,5 Liter Eiswasser verrührt und dreimal mit Dichlormethan extrahiert; die vereinigten organischen Phasen werden über Natriumsulfat getrocknet und eingedampft.

Man erhält 371,4 g (93,6% der Theorie) 4-Ethyl-3-trifluormethyl-5-methylthio-4H-1,2,4-triazol als Öl.

¹H-NMR (CDCl₃, d): 1,40-1,45; 280; 4,04-4,10 ppm.

Analog können beispielsweise auch die in der nachstehenden Tabelle 3 aufgeführten Verbindungen der Formel (III) hergestellt werden.

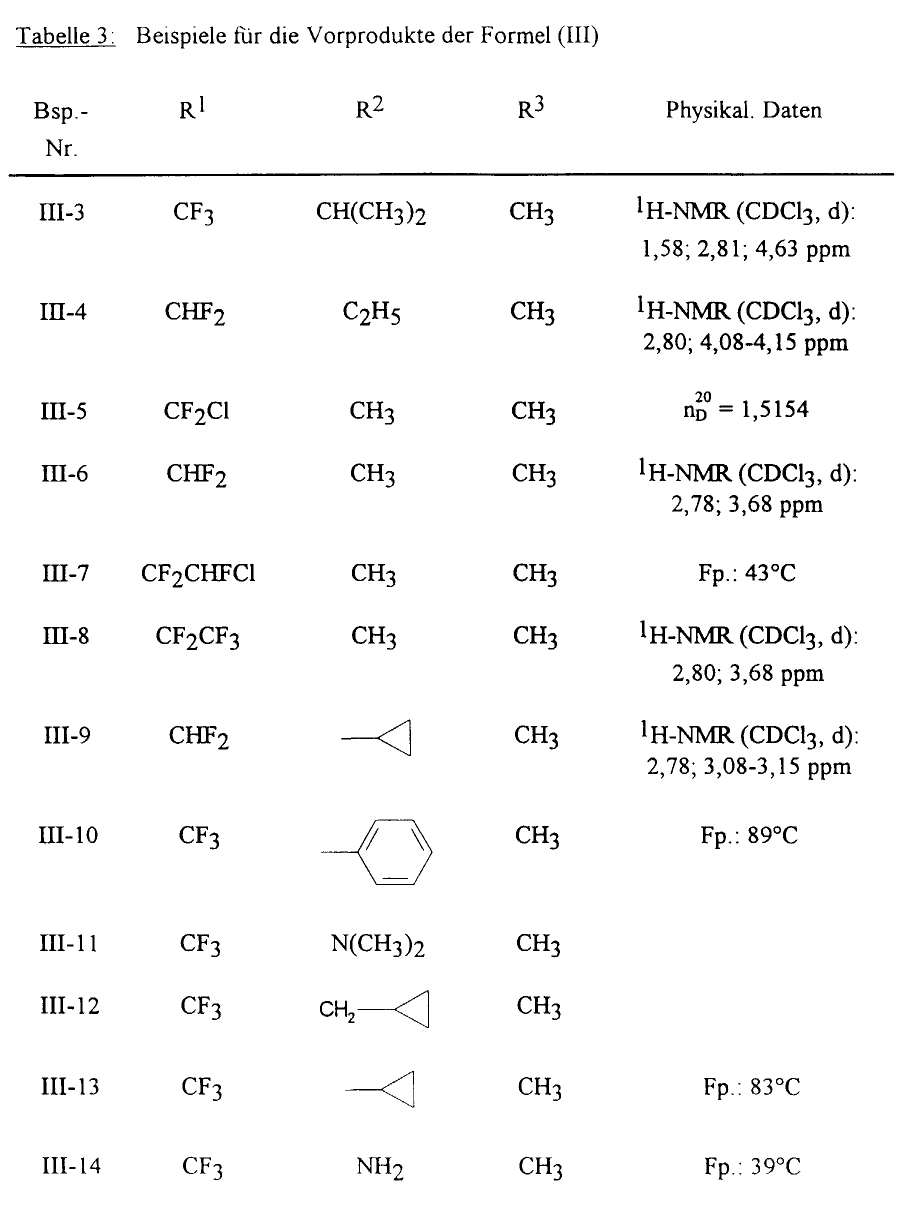

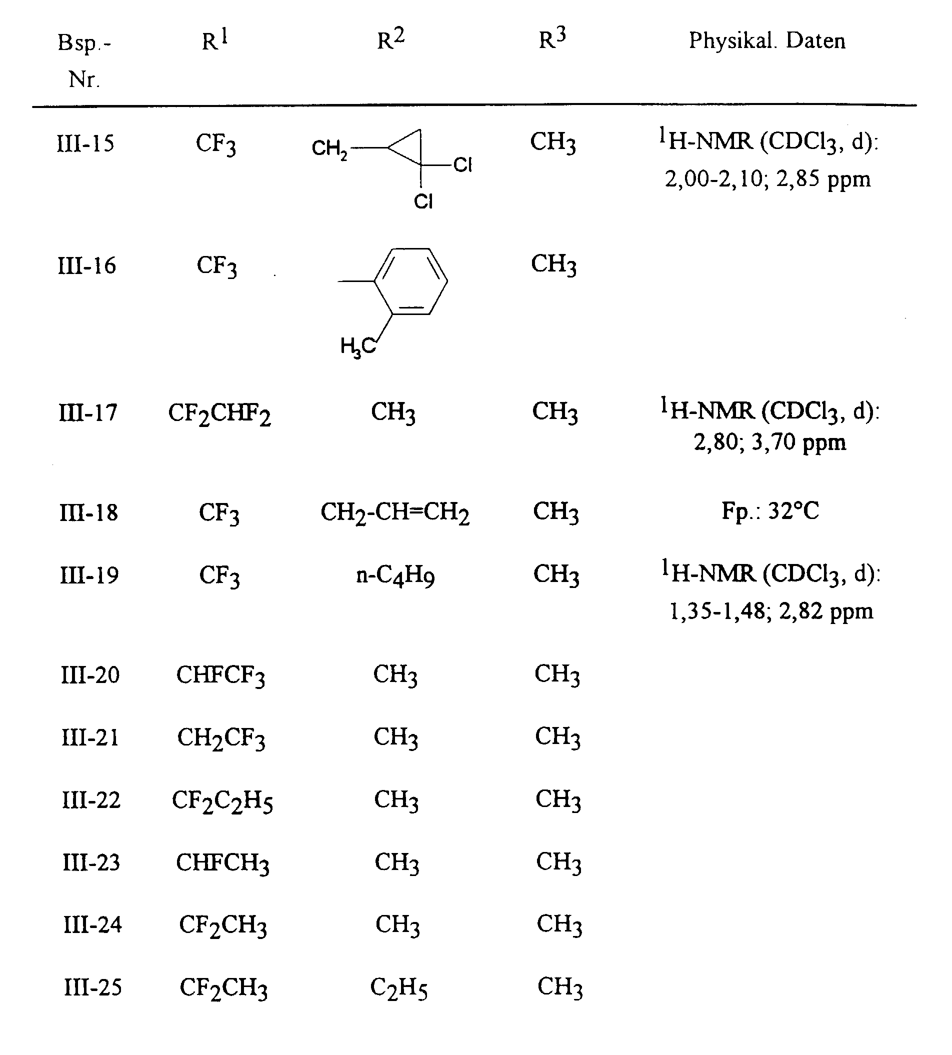

### Vorprodukte der Formel (IV):

### Beispiel (IV-1)

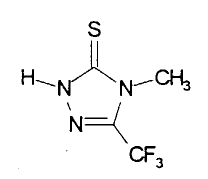

257 g (2,44 Mol) 4-Methyl-thiosemicarbazid werden zu 1,2 Liter Trifluoressigsäure gegeben und das Gemisch wird 16 Stunden unter Rückfluß erhitzt. Dann wird im Wasserstrahlvakuum eingeengt, der Rückstand mit Diethylether/Petrolether (Vol.: 5:100) verrieben und das kristallin erhaltene Produkt durch Absaugen isoliert.

Man erhält 444,5 g (95,5% der Theorie) 4-Methyl-5-trifluormethyl-2,4-dihydro-3H-1,2,4-triazol-3-thion vom Schmelzpunkt 115°C.

### Beispiel (IV-2)

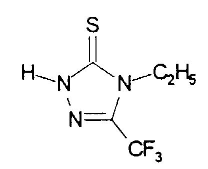

Zu 235,4 g (1,98 Mol) 4-Ethyl-thiosemicarbazid in 1000 ml Xylol werden 248 g (2,18 Mol) Trifluoressigsäure getropft, wobei die Temperatur auf 50°C ansteigt. Nach 1 Stunde bei Rückflußtemperatur wird nun das entstandene Reaktionswasser mittels Wasserabscheider innerhalb von 3 Stunden unter Rückfluß abgetrennt. Nach beendeter Reaktion wird die Reaktionsmischung eingeengt.

Man erhält 371,3 g 4-Ethyl-5-trifluormethyl-2,4-dihydro-3H-1,2,4-triazol-3-thion vom Schmelzpunkt 93°C.

Analog können beispielsweise auch die in der nachstehenden Tabelle 4 aufgeführten Verbindungen der Formel (IV) hergestellt werden.

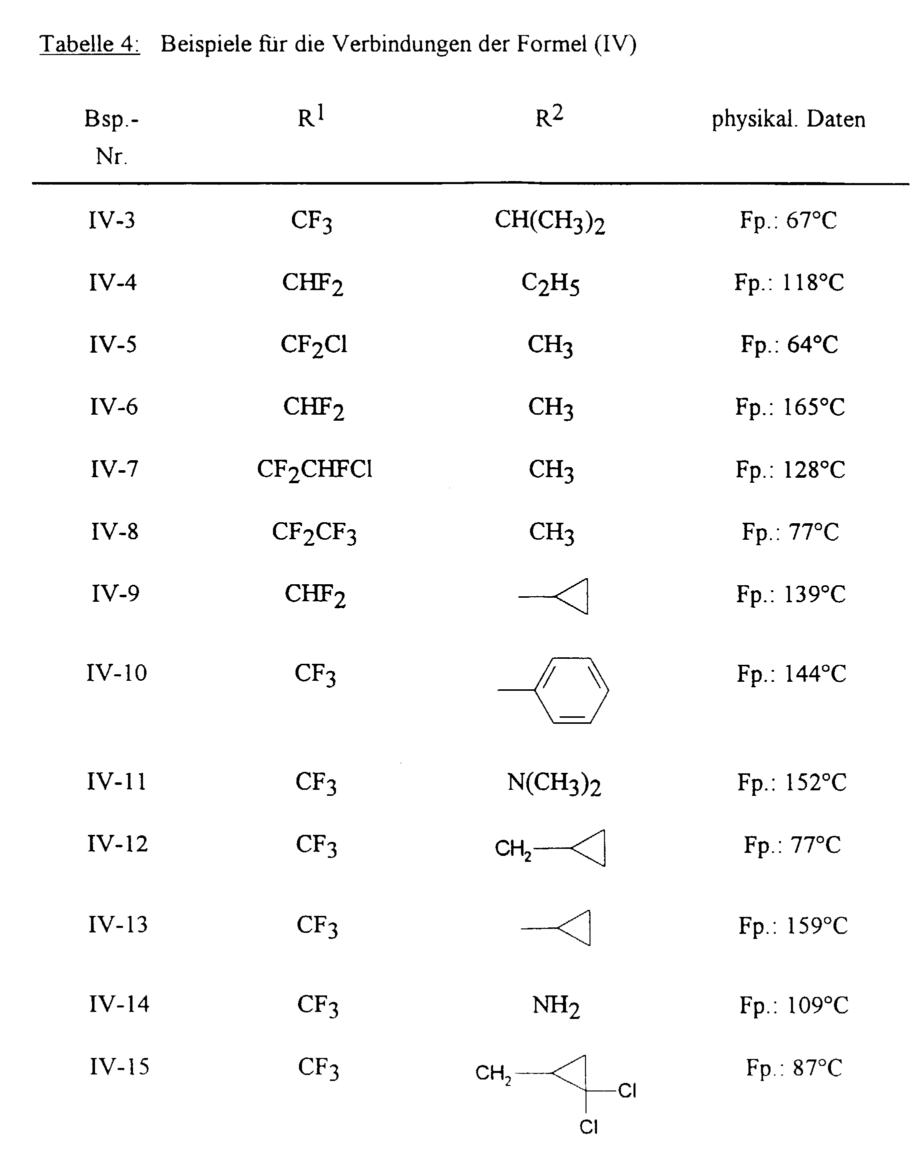

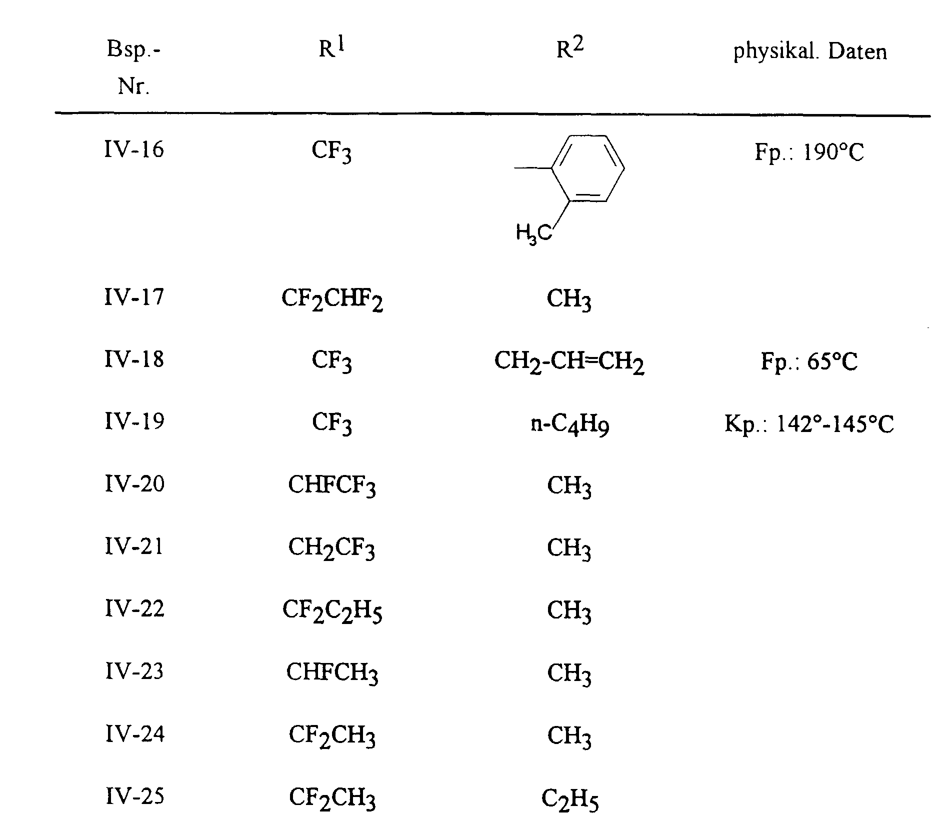

### Vorprodukte der Formel (VI):

### Beispiel (VI-1)

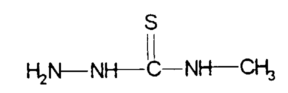

30 g (0,6 Mol) Hydrazinhydrat werden in 250 ml Ethanol auf Rückfluß erhitzt und dabei innerhalb von 30 Minuten tropfenweise mit einer Lösung von 36,5 g (0,5 Mol) Methylisothiocyanat in 50 ml Ethanol versetzt. Die Mischung wird noch 10 Minuten unter Rückfluß gerührt, dann auf+5°C abgekühlt und abgesaugt.

Man erhält 45 g (86% der Theorie) 4-Methyl-thiosemicarbazid vom Schmelzpunkt 138°C.

### Beispiel (VI-2)

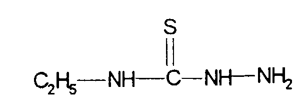

1810 g (3,62 Mol) 10%ige Hydrazinhydrat-Wasser-Lösung werden vorgelegt und unter Rühren auf 0-5°C abgekühlt. Innerhalb einer Stunde tropft man 300 g (3,45 Mol) Ethylisothiocyanat bei 0-5°C zu, rührt 2 Stunden nach und saugt das ausgefallene Produkt ab. Nach Eindampfen des Filtrats werden beide Rückstände vereinigt.

Man erhält 379 g 4-Ethyl-thiosemicarbazid vom Schmelzpunkt 85°C.

## Patentansprüche

1. Verfahren zur Herstellung von Triazolinonen der allgemeinen Formel (I) 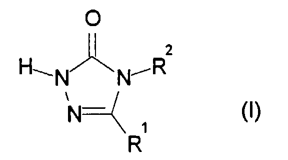 in welcher
R¹ für durch Halogen, Cyano oder C₁-C₄-Alkoxy substituiertes Alkyl mit 1 bis 6 Kohlenstoffatomen steht und
R² für Amino, für jeweils gegebenenfalls durch Halogen, Cyano oder C₁-C₄-Alkoxy substituiertes Alkyl, Alkenyl, Alkinyl, Alkoxy, Alkylamino oder Dialkylamino mit jeweils bis zu 6 Kohlenstoffatomen in den Alkyl-, Alkenyl- oder Alkinylgruppen oder für jeweils gegebenenfalls durch Halogen, Cyano, C₁-C₄-Alkyl, C₁-C₄-Alkoxy oder C₁-C₄-Alkoxycarbonyl substituiertes C₃-C₆-Cycloalkyl, C₃-C₆-Cycloalkyl-C₁-C₂-alkyl oder Phenyl steht,
dadurch gekennzeichnet, daß man
Alkylsulfonyltriazolderivate der allgemeinen Formel (II) 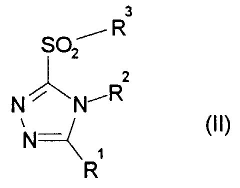 in welcher
R¹ und R² die oben angegebenen Bedeutungen haben und
R³ für Alkyl mit 1 bis 4 Kohlenstoffatomen steht,
mit wässriger Alkalilauge bei Temperaturen zwischen 0°C und 100°C bei Normaldruck umsetzt und nach Ansäuern auf übliche Weise aufarbeitet.

2. Verfahren gemäß Anspruch 1, dadurch gekennzeichnet, daß
R¹ für jeweils durch Fluor, Chlor, Brom, Cyano, Methoxy oder Ethoxy substituiertes Methyl, Ethyl, n- oder i-Propyl, n-, i- oder s-Butyl steht und
R² für Amino, für jeweils gegebenenfalls durch Fluor, Chlor, Cyano, Methoxy oder Ethoxy substituiertes Methyl, Ethyl, n- oder i-Propyl, n-, i- oder s-Butyl, Allyl.Propargyl, Methoxy, Ethoxy, n- oder i-Propoxy, n-, i- oder s-Butoxy, Methylamino, Ethylamino, n- oder i-Propylamino, n-, i- oder s-Butylamino, Dimethylamino oder Diethylamino oder für jeweils gegebenenfalls durch Fluor, Chlor, Brom, Cyano, Methyl, Ethyl, n- oder i-Propyl, Methoxy-carbonyl oder Ethoxy-carbonyl substituiertes Cyclopropyl, Cyclobutyl, Cyclopentyl, Cyclohexyl oder Phenyl steht.

3. Verfahren gemäß Anspruch 1, dadurch gekennzeichnet, daß
R¹ für jeweils entsprechend einfach, zweifach, dreifach, vierfach, fünffach, sechsfach oder siebenfach durch Fluor und/oder Chlor substituiertes Methyl, Ethyl, n- oder i-Propyl steht und
R² für Methyl, Ethyl, n- oder i-Propyl, Methoxy, Ethoxy, Methylamino, Ethylamino, Dimethylamino, Cyclopropyl, Phenyl oder Tolyl steht.

4. Verfahren gemäß Anspruch 1, dadurch gekennzeichnet, daß man die Umsetzung bei Temperaturen zwischen 10°C und 80°C durchführt.

5. Verfahren gemäß Anspruch 1, dadurch gekennzeichnet, daß man pro Mol des Alkylsulfonyltriazolderivates der Formel (II) im allgemeinen 1 bis 15 Mol eines in Wasser gelösten Alkalimetallhydroxids einsetzt.

6. Alkylsulfonyltriazolderivate der allgemeinen Formel (II) 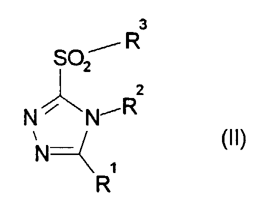 in welcher
R¹ für durch Halogen, Cyano oder C₁-C₄-Alkoxy substituiertes Alkyl mit 1 bis 6 Kohlenstoffatomen steht und
R² für Amino, für jeweils gegebenenfalls durch Halogen, Cyano oder C₁-C₄-Alkoxy substituiertes Alkyl, Alkenyl, Alkinyl, Alkoxy, Alkylamino oder Dialkylamino mit jeweils bis zu 6 Kohlenstoffatomen in den Alkyl-, Alkenyl- oder Alkinylgruppen oder für jeweils gegebenenfalls durch Halogen, Cyano, C₁-C₄-Alkyl, C₁-C₄-Alkoxy oder C₁-C₄-Alkoxycarbonyl substituiertes C₃-C₆-Cycloalkyl, C₃-C₆-Cycloalkyl-C₁-C₂-alkyl oder Phenyl steht und
R³ für Alkyl mit 1 bis 4 Kohlenstoffatomen steht.

7. Verfahren zur Herstellung der Alkylsulfonyltriazolderivate der allgemeinen Formel (II) 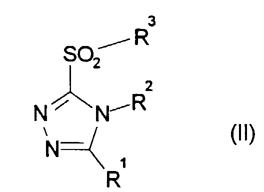 in welcher R¹, R² und R³ die in Anspruch 6 genannten Bedeutungen haben,
dadurch gekennzeichnet, daß man Alkylthiotriazolderivate der allgemeinen Formel (III) 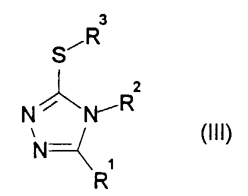 in welcher
R¹, R² und R³ die oben angegebenen Bedeutungen haben,
mit Oxidationsmitteln, gegebenenfalls in Gegenwart von Reaktionshilfsmitteln und gegebenenfalls in Gegenwart von Verdünnungsmitteln, bei Temperaturen zwischen 0°C und 100°C umsetzt.

8. Alkylthiotriazolderivate der allgemeinen Formel (III) 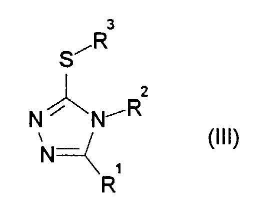 in welcher R¹, R² und R³ die in Anspruch 7 genannten Bedeutunge haben,
ausgenommen die Verbindung 4-Methyl-3 -trifluormethyl-5-methylthio-1H-1,2,4-triazol.

9. Verfahren zur Herstellung von Alkylthiotriazolderivaten der allgemeinen Formel (III) 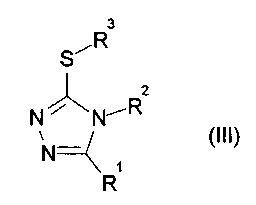 in welcher R¹, R² und R³ die in Anspruch 8 genannten Bedeutungen haben,
ausgenommen die Verbindung 4-Methyl-3-trifluormethyl-5-methylthio-, 1H-1,2,4-triazol,
dadurch gekennzeichnet, daß man
Triazolinthione der allgemeinen Formel (IV) 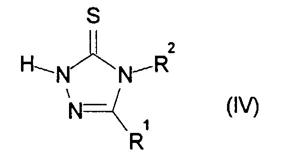 in welcher
R¹ und R² die oben angegebenen Bedeutungen haben,
mit Alkylierungsmitteln, wie z.B. Methylbromid, Methyliodid, Ethylbromid oder Ethyliodid, gegebenenfalls in Gegenwart eines Säureakzeptors, wie z.B. Natrium- oder Kalium-hydroxid, und gegebenenfalls in Gegenwart eines Verdünnungsmittels, wie z.B. Methanol oder Ethanol, bei Temperaturen zwischen 0°C und 100°C umsetzt.

## Claims

1. Process for the preparation of triazolinones of the general formula (I) 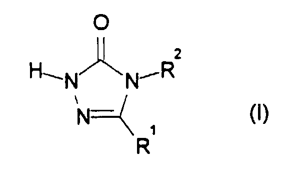 in which
R¹ represents alkyl having 1 to 6 carbon atoms which is substituted by halogen, cyano or C₁-C₄-alkoxy, and
R² represents amino, or represents alkyl, alkenyl, alkinyl, alkoxy, alkylamino or dialkylamino, each of which has up to 6 carbon atoms in the alkyl, alkenyl or alkinyl groups and each of which is optionally substituted by halogen, cyano or C₁-C₄-alkoxy, or represents C₃-C₆-cycloalkyl, C₃-C₆-cycloalkyl-C₁-C₂-alkyl or phenyl, each of which is optionally substituted by halogen, cyano, C₁-C₄-alkyl, C₁-C₄-alkoxy or C₁-C₄-alkoxy-carbonyl,
characterized in that
alkylsulphonyltriazole derivatives of the general formula (II) 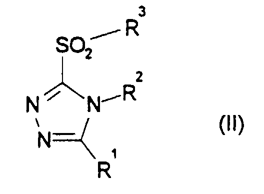 in which
R¹ and R² have the abovementioned meanings and
R³ represents alkyl having 1 to 4 carbon atoms,
are reacted with aqueous alkali metal hydroxide solution at temperatures between 0°C and 100°C under atmospheric pressure and the product is acidified and then worked up in the customary manner.

2. Process according to Claim 1, characterized in that
R¹ represents methyl, ethyl, n- or i-propyl or n-, i- or s-butyl, each of which is substituted by fluorine, chlorine, bromine, cyano, methoxy or ethoxy, and
R² represents amino, or represents methyl, ethyl, n- or i-propyl, n-, i- or s-butyl, allyl, propargyl, methoxy, ethoxy, n- or i-propoxy, n-, i- or s-butoxy, methylamino, ethylamino, n- or i-propylamino, n-, i- or s-butylamino, dimethylamino or diethylamino, each of which is optionally substituted by fluorine, chlorine, cyano, methoxy or ethoxy, or represents cyclopropyl, cyclobutyl, cyclopentyl, cyclohexyl or phenyl, each of which is optionally substituted by fluorine, chlorine, bromine, cyano, methyl, ethyl, n- or i-propyl, methoxy-carbonyl or ethoxy-carbonyl.

3. Process according to Claim 1, characterized in that
R¹ represents methyl, ethyl or n- or i-propyl, each of which is mono-, di-, tri-, tetra-, penta-, hexa- or heptasubstituted, as appropriate, with fluorine and/or chlorine, and
R² represents methyl, ethyl, n- or i-propyl, methoxy, ethoxy, methylamino, ethylamino, dimethylamino, cyclopropyl, phenyl or tolyl.

4. Process according to Claim 1, characterized in that the reaction is carried out at temperatures of between 10°C and 80°C.

5. Process according to Claim 1, characterized in that 1 to 15 mol of an alkali metal hydroxide dissolved in water are generally employed per mole of the alkylsulphonyltriazole derivative of the formula (II).

6. Alkylsulphonyltriazole derivatives of the general formula (II) 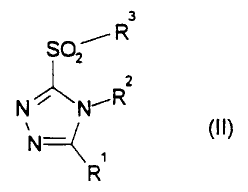 in which
R¹ represents alkyl having 1 to 6 carbon atoms which is substituted by halogen, cyano or C₁-C₄-alkoxy, and
R² represents amino, or represents alkyl, alkenyl, alkinyl, alkoxy, alkylamino or dialkylamino, each of which has up to 6 carbon atoms in the alkyl, alkenyl or alkinyl groups and each of which is optionally substituted by halogen, cyano or C₁-C₄-alkoxy, or represents C₃-C₆-cycloalkyl, C₃-C₆-cycloalkyl-C₁-C₂-alkyl or phenyl, each of which is optionally substituted by halogen, cyano, C₁-C₄-alkyl, 1-C₄-alkoxy or C₁-C₄-alkoxy-carbonyl, and
R³ represents alkyl having 1 to 4 carbon atoms.

7. Process for the preparation of the alkylsulphonyltriazole derivatives of the general formula (II) 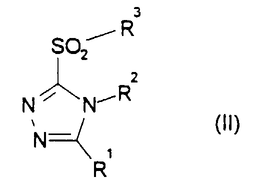 in which R¹, R² and R³ have the meanings mentioned in Claim 6,
characterized in that alkylthiotriazole derivatives of the general formula (III) 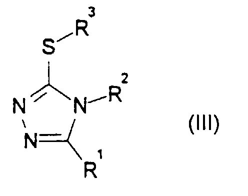 in which
R¹, R² and R³ have the abovementioned meanings,
are reacted with oxidants at temperatures between 0°C and 100°C, if appropriate in the presence of reaction auxiliaries and, if appropriate, in the presence of diluents.

8. Alkylthiotriazole derivatives of the general formula (III) 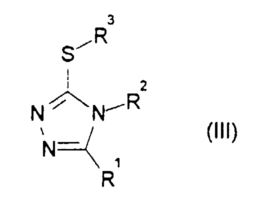 in which R¹, R² and R³ have the meanings mentioned in Claim 7,
with the exception of the compound 4-methyl-3-trifluoromethyl-5-methylthio-1H-1,2,4-triazole.

9. Process for the preparation of alkylthiotriazole derivatives of the general formula (III) 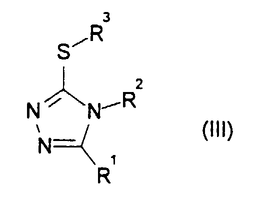 in which R¹, R² and R³ have the meanings mentioned in Claim 8,
with the exception of the compound 4-methyl-3-trifluoromethyl-5-methylthio-1H-1,2,4-triazole,
characterized in that
triazolinethiones of the general formula (IV) 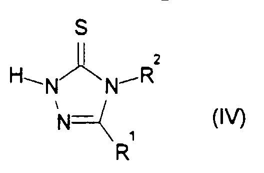 in which
R¹ and R² have the abovementioned meanings, are reacted with alkylating agents, such as, for example, methyl bromide, methyl iodide, ethyl bromide or ethyl iodide, at temperatures between 0°C and 100°C, if appropriate in the presence of an acid acceptor, such as, for example, sodium hydroxide or potassium hydroxide, and, if appropriate, in the presence of a diluent, such as, for example, methanol or ethanol.

## Revendications

1. Procédé pour la préparation de triazolinones répondant à la formule générale (I) 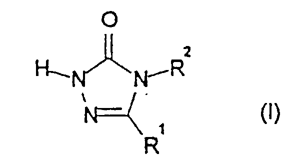 dans laquelle
R¹ représente un groupe alkyle contenant de 1 à 6 atomes de carbone, portant un ou plusieurs substituants identiques ou différents halogéno, cyano ou alcoxy en C₁-C₄, et
R² représente un groupe amino; un groupe alkyle, un groupe alcényle, un groupe alcynyle, un groupe alcoxy, un groupe alkylamino ou un groupe dialkylamino contenant respectivement jusqu'à 6 atomes de carbone dans les groupes alkyle, alcényle ou alcynyle, chacun de ces groupes portant éventuellement un ou plusieurs substituants identiques ou différents halogéno, cyano ou alcoxy en C₁-C₄; ou représente un groupe cycloalkyle en C₃-C₆, un groupe cycloalkyl(en C₃-C₆)alkyle en C₁-C₂ ou encore un groupe phényle, chacun de ces groupes portant éventuellement un ou plusieurs substituants identiques ou différents halogéno, cyano, alkyle en C₁-C₄, alcoxy en C₁-C₄ ou alcoxy(en C₁-C₄)carbonyle,
caractérisé en ce que
on fait réagir des dérivés d'alkylsulfonyltriazoles répondant à la formule générale (II) 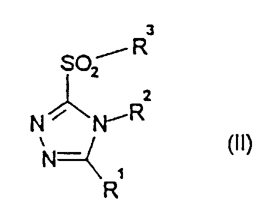 dans laquelle
R¹ et R² ont les significations indiquées ci-dessus et
R³ représente un groupe alkyle contenant de 1 à 4 atomes de carbone,
avec de la lessive alcaline aqueuse, à des températures entre 0°C et 100°C, sous pression normale et, après acidification, on traite de la manière habituelle.

2. Procédé selon la revendication 1, caractérisé en ce que
R¹ représente un groupe méthyle, un groupe éthyle, un groupe n- ou i-propyle, un groupe n-, i- ou s-butyle, chacun de ces groupes portant un ou plusieurs substituants identiques ou différents fluoro, chloro, bromo, cyano, méthoxy ou éthoxy,
R² représente un groupe amino; un groupe méthyle, un groupe éthyle, un groupe n- ou i-propyle, un groupe n-, i- ou s-butyle, un groupe allyle, un groupe propargyle, un groupe méthoxy, un groupe éthoxy, un groupe n- ou i- propoxy, un groupe n-, i- ou s-butoxy, un groupe méthylamino, un groupe éthylamino, un groupe n- ou 1-propylamino, un groupe n, i- ou s-butylamino, un groupe diméthylamino ou un groupe diéthylamino, chacun de ces groupes portant éventuellement un ou plusieurs substituants identiques ou différents fluoro, chloro, cyano, méthoxy ou éthoxy; ou représente un groupe cyclopropyle, un groupe cyclobutyle, un groupe cyclopentyle, un groupe cyclohexyle ou un groupe phényle, chacun de ces groupes portant éventuellement un ou plusieurs substituants identiques ou différents fluoro, chloro, bromo, cyano, méthyle, éthyle, n- ou i-propyle, méthoxycarbonyle ou éthoxy-carbonyle.

3. Procédé selon la revendication 1, caractérisé en ce que
R¹ représente un groupe méthyle, un groupe éthyle, un groupe n- ou i-propyle, chacun de ces groupes portant, de manière correspondante, un, deux, trois, quatre, cinq, six ou sept substituants fluoro et/ou chloro identiques ou différents,
R² représente un groupe méthyle, un groupe éthyle, un groupe n- ou i-propyle, un groupe méthoxy, un groupe éthoxy, un groupe méthylamino, un groupe éthylamino, un groupe diméthylamino, un groupe cyclopropyle, un groupe phényle ou un groupe tolyle.

4. Procédé selon la revendication 1, caractérisé en ce qu'on effectue la mise en réaction à des températures entre 10°C et 80°C.

5. Procédé selon la revendication 1, caractérisé en ce qu'on met en oeuvre, par mole du dérivé d'alkylsulfonyltriazole de formule (II), en général de 1 à 15 moles d'un hydroxyde de métal alcalin dissous dans de l'eau.

6. Dérivés d'alkylsulfonyltriazoles répondant à la formule générale (II) 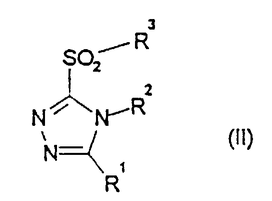 dans laquelle
R¹ représente un groupe alkyle contenant de 1 à 6 atomes de carbone, portant un ou plusieurs substituants identiques ou différents halogéno, cyano ou alcoxy en C₁-C₄, et
R² représente un groupe amino; un groupe alkyle, un groupe alcényle, un groupe alcynyle, un groupe alcoxy, un groupe alkylamino ou un groupe dialkylamino contenant respectivement jusqu'à 6 atomes de carbone dans les groupes alkyle, alcényle ou alcynyle, chacun de ces groupes portant éventuellement un ou plusieurs substituants identiques ou différents halogéno, cyano ou alcoxy en C₁-C₄; ou encore un groupe cycloalkyle en C₃-C₆, un groupe cycloalkyl(en C₃-C₆)alkyle en C₁-C₂ ou encore un groupe phényle, chacun de ces groupes portant éventuellement un ou plusieurs substituants identiques ou différents halogéno, cyano, alkyle en C₁-C₄, alcoxy en C₁-C₄ ou alcoxy(en C₁-C₄)carbonyle, et
R³ représente un groupe alkyle contenant de 1 à 4 atomes de carbone.

7. Procédé pour la préparation des dérivés d'alkylsulfonyltriazoles répondant à la formule générale (II) 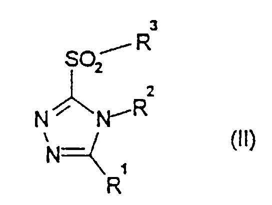 dans laquelle
R¹, R² et R³ ont les significations mentionnées à la revendication 6,
caractérisé en ce qu'on fait réagir des dérivés d'alkylthiotriazoles répondant à la formule générale (III) 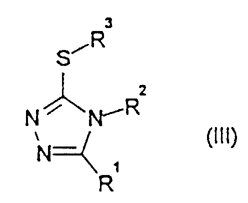 dans laquelle
R¹, R² et R³ ont les significations indiquées ci-dessus,
avec des agents d'oxydation, éventuellement en présence d'adjuvants réactionnels et éventuellement en présence de diluants, à des températures entre 0°C et 100°C.

8. Dérivés d'alkylthiotriazoles répondant à la formule générale (III) 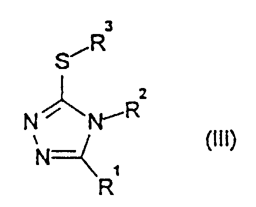 dans laquelle
R¹, R² et R³ ont les significations mentionnées à la revendication 7,
à l'exception du composé 4-méthyl-3-trifluorométhyl-5-méthylthio-1H-1,2,4-triazole.

9. Procédé pour la préparation de dérivés d'alkylthiotriazoles répondant à la formule générale (III) 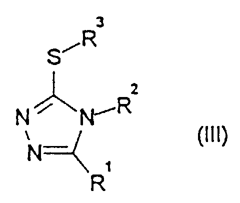 dans laquelle
R¹, R² et R³ ont les significations mentionnées à la revendication 8,
à l'exception du composé 4-méthyl-3-trifluorométhyl-5-méthylthio-1H-1,2,4-triazole,
caractérisé en ce qu'on fait réagir des triazolinethiones répondant à la formule générale (IV) 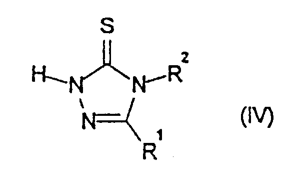 dans laquelle
R¹ et R² ont les significations indiquées ci-dessus,
avec des agents d'alkylation tels que par exemple le bromure de méthyle, l'iodure de méthyle, le bromure d'éthyle ou l'iodure d'éthyle, éventuellement en présence d'un agent neutralisant les acides, tel que par exemple l'hydroxyde de sodium ou de potassium, et éventuellement en présence d'un diluant tel que par exemple le méthanol ou l'éthanol, à des températures entre 0°C et 100°C.
